# DEMANDE DE BREVET EUROPEEN

(11) **EP 1 655 020 A1**
(43) Date de publication de la demande: **10.05.2006**
(21) Numéro de dépôt: 05300893.4
(22) Date de dépôt: 04.11.2005
(51) Int. Cl.: A61K 8/55, A61K 8/37, A61Q 1/06

(54) **Composition cosmétique anhydre apte à former un organogel**

(30) Priorité: 05.11.2004 FR 0452539
(71) Demandeur: L'OREAL, 75008 Paris (FR)
(72) Inventeur: Blin, Xavier, 75015 Paris (FR)
(74) Mandataire: Le Coupanec, Pascale A.M.P.

(57) **Abrégé**

La présente invention concerne une composition cosmétique anhydre contenant, dans un milieu physiologiquement acceptable, au moins une phase grasse comprenant au moins un ester hydrocarboné court et au moins un glycérophospholipide non hydrogéné, ledit ester hydrocarboné court et ledit glycérophospholipide non hydrogéné y étant respectivement présents en des teneurs correspondant à celles requises dans un mélange binaire constitué desdits glycérophospholipide non hydrogéné et ester hydrocarboné court pour lui conférer une aptitude à former un organogel par mise en contact avec un milieu aqueux.

## Description

La présente invention concerne une composition cosmétique anhydre destinée au soin et/ou au maquillage et apte à former un organogel au contact d'un milieu aqueux.

Les compositions cosmétiques visées par la présente invention sont, plus particulièrement, des produits de maquillage et/ou de soin destinées à être appliquées sur les matières kératiniques, notamment la peau, les lèvres et/ou les phanères, et peuvent être formulées notamment en rouge à lèvres, en baume à lèvres, en crayon à lèvres, en fond de teint liquide ou solide, notamment coulé en stick ou en coupelle, en produit anti-cernes et en produit de coloration de la peau, en tatouage éphémère, en produit de maquillage des yeux, comme les eye-liners, en particulier sous forme de crayon, et en mascaras notamment sous forme de pain ou encore de fard à paupières.

La tenue d'une composition cosmétique, et notamment la tenue de sa couleur, en particulier dans le cadre d'un rouge à lèvres, est une des caractéristiques déterminantes dans l'obtention d'un effet esthétique. Celle-ci est particulièrement difficile à obtenir et fait l'objet de nombreuses et actives recherches. Il est en particulier difficile d'arriver à concilier au sein d'une composition cosmétique de bonnes propriétés de tenue avec une brillance satisfaisante. Par ailleurs, outre sa capacité à conférer un aspect esthétique, une composition cosmétique se doit également d'être agréable à appliquer et à porter, et ne doit pas provoquer de sensation collante et/ou de sensation de gras et/ou de sensation de dessèchement.

Afin d'améliorer la tenue et le maintien d'un dépôt homogène d'une composition cosmétique, il a notamment été proposé d'utiliser des éléments volatils dans la formulation de ces compositions. L'évaporation de ces éléments se traduit par une concentration du dépôt de la composition, entraînant une réduction de la sensibilité à l'usure de ce dépôt. Cependant, ces compositions présentent l'inconvénient de provoquer une sensation de dessèchement.

Une autre alternative a consisté à structurer le dépôt de la composition cosmétique sous forme d'un gel, en particulier d'un hydrogel. Il a ainsi été développé des rouges à lèvres comprenant une association de tensioactifs siliconés susceptible de former un gel aqueux par mise en contact avec de l'eau (apportée par la salive) se traduisant ainsi par un épaississement du dépôt. Toutefois, le gel ainsi formé demeure sensible à l'eau.

D'autres structures de type gel consistant en deux phases non miscibles (de type eau-dans-huile), susceptibles de contenir des agents actifs hydrophiles, ont été décrites dans le brevet US 6 342 238. Elles sont gélifiées et stabilisées au moyen d'une lécithine, généralement hydrogénée.

La lécithine est un mélange de composés contenant à titre d'élément majoritaire de la phosphatidylcholine, molécule appartenant à la famille des glycérophospholipides.

La lécithine est usuellement utilisée en faible proportion (en général inférieure à 10 %) comme agent tensioactif pour la préparation de compositions cosmétiques sous forme d'émulsion aqueuse ou anhydre, par exemple des rouges à lèvres (voir notamment WO 02/098378, WO 02/098379, WO 98/52927, US 2002/0048597 et EP 0 062 896).

Elle a également été proposée, seule ou en combinaison avec d'autres tensioactifs, à des fins de structuration, sous forme de micelles inverses et de structures de cristaux liquides lyotropes, de compositions anhydres comprenant une phase lipophile et un solvant polaire ou un mélange de solvants polaires, comme le décrivent les documents US 6 325 995 et WO 95/11000. Les solvants polaires décrits dans ces documents sont par nature des solvants hydrophiles.

Les documents EP 0 534 823 et WO 02/47617 l'utilisent pour formuler des compositions cosmétiques anhydres dont l'application sur la peau ou les lèvres se traduit par la formation spontanée, en présence d'un milieu aqueux, de vésicules lipidiques, ou liposomes, favorisant la libération et l'activité d'actifs cosmétiques et/ou dermopharmaceutiques à partir de ces compositions.

Cependant, ces compositions cosmétiques ne permettent pas de concilier tenue de la couleur améliorée et brillance satisfaisante. Par ailleurs, leur application, notamment sur les lèvres, se traduit par une sensation collante et de gras, voire également de dessèchement.

La présente invention a précisément pour but de fournir une composition de soin et/ou de maquillage des matières kératiniques, comme la peau et/ou les lèvres, visant à donner satisfaction à l'ensemble des exigences mentionnées précédemment.

Les inventeurs ont ainsi découvert qu'il était possible d'utiliser au moins un glycérophospholipide non hydrogéné associé à un solvant organique particulier pour formuler une composition cosmétique anhydre apte à former un organogel par mise en contact avec un milieu aqueux.

Ainsi, selon un de ses premiers aspects, la présente invention a pour objet une composition cosmétique anhydre contenant, dans un milieu physiologiquement acceptable, au moins une phase grasse comprenant au moins un ester hydrocarboné court et au moins un glycérophospholipide non hydrogéné, l'ester hydrocarboné court et le glycérophospholipide non hydrogéné y étant respectivement présents en des teneurs correspondant à celles requises dans un mélange binaire constitué desdits glycérophospholipide non hydrogéné et ester court pour lui conférer une aptitude à former un organogel par mise en contact avec un milieu aqueux.

Les compositions cosmétiques comprenant un mélange binaire de cette nature présentent également une aptitude à former un organogel par mise en contact avec un milieu aqueux.

Au sens de la présente invention, l'expression "glycérophospholipide non hydrogéné" est utilisée pour désigner un glycérophospholipide comprenant au moins un acide gras comprenant au moins une insaturation.

Au sens de la présente invention, on entend par "teneur requise", la teneur minimale d'un composant nécessaire à l'obtention de l'effet attendu, à savoir dans le cas du glycérophospholipide non hydrogéné et de l'ester hydrocarboné court, l'obtention d'un organogel par mise en contact avec un milieu aqueux.

Au sens de la présente invention, on entend par "milieu aqueux", un milieu possédant une teneur en eau suffisante pour transformer en organogel, soit la composition cosmétique selon l'invention, appliquée sur un support à maquiller, soit un mélange binaire glycérophospholipide non hydrogéné ― ester hydrocarboné court. Ce milieu aqueux peut être fourni par exemple par la sueur, la salive, les larmes ou tout autre liquide naturel ou artificiel, comme l'eau, une émulsion eau-dans-huile ou une lotion, susceptible d'être disposée sur le support à maquiller avant ou après application d'une composition cosmétique conforme à l'invention.

La formation de l'organogel, suite à un contact de la composition avec un milieu aqueux, comme la salive, la sueur ou l'eau, au niveau du support considéré, conduit avantageusement à la formation d'un dépôt présentant une tenue améliorée et une brillance satisfaisante. Par ailleurs, ce dépôt se traduit également par une sensation de douceur et de confort agréable, avec une sensation collante et/ou une sensation de gras sensiblement réduite, voire absente.

Selon un autre de ses aspects, la présente invention a pour objet une composition cosmétique anhydre contenant, dans un milieu physiologiquement acceptable, au moins une phase grasse comprenant au moins un ester hydrocarboné court et au moins un glycérophospholipide non hydrogéné, ladite composition étant exempte de solvant polaire hydrophile.

Au sens de la présente invention, on entend désigner par "solvant polaire hydrophile" un solvant soluble dans l'eau à température ambiante (25 °C) et à une concentration supérieure à 5% en volume, par exemple un solvant hydrocarbonée comprenant au moins une fonction alcool notamment l'éthanol, l'isopropanol, l'hexanol, le propanol, l'alcool benzylique, le propylène glycol, le polypropylène glycol, le butylène glycol, le maltitol, le sorbitol, la glycérine, et leurs mélanges.

Selon un autre de ses aspects, la présente invention a encore pour objet une composition cosmétique anhydre contenant, dans un milieu physiologiquement acceptable, au moins une phase grasse comprenant au moins un ester hydrocarboné court et au moins un glycérophospholipide non hydrogéné, ladite composition comprenant moins de 5 % de cire en poids par rapport au poids total de la composition.

Les compositions cosmétiques selon l'invention peuvent se présenter sous forme de pâte, de gel, de liquide, de crème, de semi-solide ou de solide. En particulier, les compositions cosmétiques selon l'invention sont sous forme coulée, et plus particulièrement sous la forme d'un stick.

Par "composition sous forme coulée", on entend désigner, au sens de la présente invention, une composition solide, ou semi-solide, obtenue à l'issue du refroidissement d'une composition introduite à l'état de fusion dans un moule. Les compositions peuvent être coulées sous forme d'un stick ou d'un bâton, ou dans une coupelle.

Selon un autre aspect, la présente invention a encore pour objet un rouge à lèvres anhydre contenant, dans un milieu physiologiquement acceptable, au moins une phase grasse comprenant au moins un ester hydrocarboné court et au moins un glycérophospholipide non hydrogéné, ledit rouge à lèvres étant exempt de solvant polaire hydrophile.

Selon encore un autre aspect, la présente invention a également pour objet un rouge à lèvres anhydre contenant, dans un milieu physiologiquement acceptable, au moins une phase grasse comprenant au moins un ester hydrocarboné court et au moins un glycérophospholipide non hydrogéné, ledit rouge à lèvres comprenant moins de 5 % en poids de cire par rapport au poids total de la composition.

Selon un autre de ses aspects, la présente invention a également pour objet un procédé cosmétique de soin, de maquillage et/ou de traitement des matières kératiniques comprenant au moins l'étape d'application sur ces matières kératiniques d'une composition cosmétique conforme à la présente invention.

Au sens de la présente invention, on entend désigner par "matière kératinique", l'ensemble des parties du corps humain (ou animal) contenant au moins une couche de kératine, et notamment la peau, les lèvres, les cheveux, les ongles, les sourcils et les cils.

Selon un autre aspect, la présente invention concerne également un support synthétique maquillé comprenant sur tout ou partie de sa surface au moins une couche d'une composition conforme à la présente invention.

Selon encore un autre de ses aspects, la présente invention a encore pour objet l'utilisation d'au moins un glycérophospholipide non hydrogéné en association avec au moins un ester court pour conférer à une composition cosmétique une aptitude à former un organogel par mise en contact avec un milieu aqueux.

Selon encore un autre de ses aspects, la présente invention a encore pour objet un kit de soin et/ou de maquillage comportant :
- au moins une première composition comprenant de l'eau, et
- au moins une deuxième composition anhydre contenant, dans un milieu physiologiquement acceptable, au moins une phase grasse comprenant au moins un ester hydrocarboné court et au moins un glycérophospholipide non hydrogéné.

La première composition comprend de l'eau en une quantité suffisante pour que la deuxième composition forme un organogel lorsqu'elle est mise en contact avec la première composition.

La première composition peut comprendre, éventuellement, des parfums, des colorants et/ou des actifs, notamment des actifs cosmétiques et/ou dermatologiques. La première composition peut être appliquée sur une matière kératinique, par exemple à l'aide d'un pulvérisateur, d'un aérosol, d'un flacon pompe, à la main ou à l'aide d'un mouchoir ou d'une éponge.

Les compositions cosmétiques conformes à la présente invention peuvent présenter une tenue, et notamment une tenue de la couleur, améliorée.

D'une manière générale, les compositions conformes à la présente invention peuvent présenter, également, un transfert sur les tissus et une migration dans les rides et ridules réduits.

De même, les compositions cosmétiques conformes à la présente invention peuvent présenter, généralement, également, de bonnes propriétés de maintien du dépôt au cours du temps, tout en conservant des propriétés de brillance et de confort satisfaisantes.

Par ailleurs, les compositions conformes à la présente invention peuvent également présenter, généralement, une sensation de gras et une sensation collante sensiblement réduite, voire absente.

### GLYCEROPHOSPHOLIPIDE NON HYDROGENE

Au sens de la présente invention, on entend désigner par glycérophospholipide non hydrogéné, un ester obtenu par réaction du glycérol avec au moins un acide gras insaturé et l'acide phosphorique, ledit acide phosphorique étant substitué par un composé choisi parmi les alcools portant une fonction amine, notamment un bêta-amino alcool. Le bêta-amino alcool peut être choisi, par exemple, parmi la choline, l'éthanolamine ou la sérine.

Le glycérophospholipide non hydrogéné peut notamment être défini selon la formule générale (I) suivante : 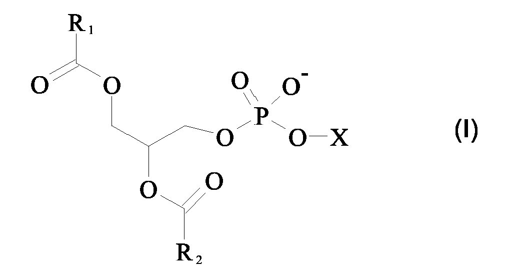 dans laquelle :
- R₁ et R₂ représentent, indépendamment l'un de l'autre, un acide gras comprenant de 4 à 24 atomes de carbone, saturé ou insaturé, le cas échéant ramifié, et susceptible d'être substitué par une ou plusieurs fonctions hydroxy et/ou amines, et
- X représente un substituant de formule générale R₃R₄R₅N⁺-CH(R₆)-CH₂-
dans lequel R₃, R₄, R₅ et R₆ représentent, indépendamment l'un de l'autre, un hydrogène, des groupes alkyles comprenant de 1 à 6 atomes de carbone, et/ou une fonction carboxy. Notamment, X peut être choisi parmi la choline, la sérine ou l'éthanolamine.

Par "insaturé" ou "insaturation", on entend désigner la présence d'au moins une, voire de plusieurs, doubles ou triples liaisons entre deux atomes de carbone.

Selon une variante de réalisation, R₁ et R₂, indépendamment l'un de l'autre, sont avantageusement choisis parmi l'acide butyrique, l'acide caproïque, l'acide caprylique, l'acide caprique, l'acide caproléïque, l'acide laurique, l'acide lauroléïque, l'acide myristique, l'acide myristoléique, l'acide palmitique, l'acide palmitoléique, l'acide stéarique, l'acide oléique, l'acide linoléique, l'acide linolénique, l'acide arachidique, l'acide isostéarique, l'acide dihydroxystéarique et l'acide ricinoléique.

Ainsi, au sens de la présente invention, on entend désigner par "glycérophospholipide", un mélange de composés de formule générale (I), dans laquelle R₁ et R₂ représentent, indépendamment l'un de l'autre, un acide gras tel que défmi précédemment, et X représente un composé azoté basique, également tel que défini ci-dessus.

Le glycérophospholipide non hydrogéné convenant à la mise en oeuvre de l'invention est notamment choisi parmi la phosphatidylcholine non hydrogénée, la phosphatidyléthanolamine non hydrogénée, la phosphatidylsérine non hydrogénée, et leurs mélanges.

Selon un mode de réalisation, le glycérophospholipide non hydrogéné est avantageusement un ester de glycérol, d'acide gras insaturé, d'acide phosphorique et de choline, également dénommé phosphatidylcholine (PC).

Le glycérophospholipide non hydrogéné convenant à la mise en oeuvre de l'invention peut provenir d'une lécithine non hydrogénée. Notamment, la lécithine non hydrogénée peut comprendre, majoritairement, en tant que glycérophospholipide non hydrogéné, de la phosphatidylcholine non hydrogénée.

La phosphatidylcholine (PC) non hydrogénée convenant à la mise en oeuvre des compositions conformes à l'invention peut être d'origine "naturelle" ou "synthétique".

La PC dite "naturelle" peut être obtenue par extraction à partir de sources animales ou végétales, comme le soja, le tournesol, ou les oeufs. La phosphatidylcholine non hydrogénée obtenue naturellement, comme par exemple à partir de soja, contient généralement comme acide gras estérifiant le glycérol, de l'acide palmitique, de l'acide stéarique, de l'acide palmitoléique, de l'acide oléique, de l'acide linoléique, de l'acide linolénique, et éventuellement des acides gras en C₂₀ à C₂₂.

La PC dite "naturelle" est avantageusement utilisée sous forme non hydrogénée, c'est-à-dire que les doubles liaisons présentes sur les acides gras n'ont pas été réduites, ou n'ont été que partiellement réduites.

Par "phosphatidylcholine synthétique", on entend désigner au sens de la présent invention, de la phosphatidylcholine comportant au moins un acide gras distinct de ceux susceptibles d'être présents dans les PC dites naturelles.

Par "PC synthétique", on entend également désigner de la PC dite naturelle soumise à des modifications, telles que l'hydrogénation partielle, c'est-à-dire que seule une fraction des doubles liaisons présentes sur les acides gras insaturés est maintenue.

Parmi les sources de phosphatidylcholine non hydrogénée plus ou moins purifiée convenant à la mise en oeuvre des compositions cosmétiques conformes à la présente invention, il peut être fait mention de EMULMETIK 930 commercialisée par la société LUCAS MEYER.

Selon un mode particulier de réalisation, les compositions cosmétiques conformes à l'invention comprennent une teneur en glycérophospholipide(s) non hydrogéné(s), d'au moins 5 %, d'au moins 10 %, en particulier d'au moins 15 %, et plus particulièrement d'au moins 20 % en poids par rapport au poids total de la composition.

Le glycérophospholipide non hydrogéné convenant à la mise en oeuvre de la présente invention peut, notamment, être introduit dans la composition sous la forme d'une lécithine non hydrogénée. Celle-ci est généralement obtenue par extraction lipidique au moyen de solvants apolaires, à partir de matières grasses végétales ou animales. Cette fraction lipidique comprend, habituellement, majoritairement, des glycérophospholipides, dont la phosphatidylcholine.

Les sources animale ou végétale utilisables pour extraire les lécithines non hydrogénées sont, par exemple, le soja, le tournesol ou les oeufs. Les glycérophospholipides compris, en proportion élevée, dans ces lécithines sont principalement la phosphatidylcholine et la phosphatidyléthanolamine.

Les lécithines non hydrogénées convenant à la mise en oeuvre de la présente invention peuvent être des lécithines issues du soja, du tournesol, de l'oeuf et/ou leurs mélanges.

Les lécithines sont habituellement fournies sous forme dissoutes dans des acides gras, des triglycérides ou d'autres solvants, ou sous forme de poudres ou de pains.

Ce sont usuellement des mélanges de lécithines, dont la teneur en glycérophospholipides, dans les produits tels que commercialisés, varie de manière générale d'environ au moins 15 % à environ au moins 95 %.

De manière avantageuse, la lécithine non hydrogénée utilisée comme matière première pour la préparation de la composition selon l'invention comprend au moins 45 % en poids, en particulier au moins 65 % en poids, en particulier au moins 75 % en poids, en particulier au moins 85 % en poids, et plus particulièrement au moins 95 % en poids de glycérophospholipide non hydrogéné par rapport au poids total de la lécithine.

Parmi les lécithines non hydrogénées pouvant convenir à la mise en oeuvre des compositions cosmétiques conformes à la présente invention, il peut être fait mention des lécithines commercialisées sous les références NATTERMANN PHOSPHOLIPID® , PHOSPHOLIPON 80® et PHOSALE 75® par la société AMERICAN LECITHIN COMPANY, EPIKURON 145V, TOPCITHIN 300, EMULMETIK 930 et OVOTHIN 200 commercialisées par la société LUCAS MEYER.

La teneur en lécithine non hydrogénée dans les compositions cosmétiques conformes à la présente invention est ajustée par l'homme du métier de manière à ce que la teneur en glycérophospholipides non hydrogénés soit suffisante pour conférer à un mélange binaire glycérophospholipide non hydrogéné/ester hydrocarboné court une aptitude à former un organogel lors de sa mise en contact avec un milieu aqueux.

La lécithine non hydrogénée peut représenter, au moins 5% en poids, par exemple au moins 10 % en poids, en particulier au moins 15 % en poids, et plus particulièrement au moins 20 % en poids par rapport au poids total de la composition.

### ESTERS COURTS

Les compositions cosmétiques comportent au moins un ester hydrocarboné court.

Au sens de la présente invention, on entend par "ester hydrocarboné court", un ester hydrocarboné comprenant moins de 40 et plus de 10 atomes de carbone.

En particulier, les esters hydrocarbonés utilisables dans les compositions cosmétiques conformes à la présente invention comprennent plus de 12, notamment plus de 16, voire plus de 25 atomes de carbone.

Les esters hydrocarbonés courts peuvent être des monoesters, des diesters non hydroxylés et sont plus particulièrement des monoesters c'est-à-dire portant une unique fonction ester. Ces esters peuvent être linéaires, ramifiés ou cycliques, saturés ou insaturés. En particulier, ils sont ramifiés et saturés.

Lorsqu'ils sont insaturés, ils peuvent comprendre de manière avantageuse, de 1 à 4 double liaisons, et en particulier de 1 à 2 double liaisons.

Les chaînes hydrocarbonées disposées de part et d'autre de la fonction ester du monoester peuvent également comprendre une ou plusieurs fonctions hydroxy et/ou une ou plusieurs fonctions carboxy et/ou une ou plusieurs fonctions amines.

L'ester hydrocarbonée est non volatil. Au sens de la présente invention, on entend par "huile non-volatile", une huile ayant une pression de vapeur inférieure à 0,13 Pa.

En particulier, les esters hydrocarbonés utilisables dans les compositions cosmétiques conformes à la présente invention, peuvent répondre à la formule RCOOR'
dans laquelle RCOO représente un reste d'acide gras comportant de 2 à 28 atomes de carbone, et R' représente une chaîne hydrocarbonée contenant de 1 à 28 atomes de carbone.

Ces esters sont en particulier des esters non volatils, notamment, en C₁₁ à C₂₅ et en particulier en C₁₄ à C₂₂. Ils peuvent être choisis parmi des esters des acides en C₂ à C₁₈ et notamment, des alcools en C₂ à C₂₀ ou de polyols en C₂ à C₈ ou de leurs mélanges, à la condition que l'ester comprenne plus de 10 carbones.

Ainsi, les esters peuvent être choisis parmi une liste non limitative comprenant les esters de l'acide néopentanoïque comme le néopentanoate d'isodécyle, le néopentanoate d'isotridécyle, le néopentanoate d'isostéaryle, le néopentanoate d'octyldodécyle, les esters de l'acide isononanoïque comme l'isononanoate d'isononyle, l'isononanoate d'octyle, l'isononanoate d'isodécyle, l'isononanoate d'isotridécyle, l'isononanoate d'isostéaryle, mais aussi les esters de l'alcool isopropylique, tels que le myristate d'isopropyle, le palmitate d'isopropyle, le stéarate ou l'isostéarate d'isopropyle, l'octanoate de cétyle, l'octanoate de tridécyle, le 4-diheptanoate et le palmitate d'éthyle 2-hexyle, les benzoates d'alkyle, le diheptanoate de polyéthylène glycol, le diéthyl 2-d'hexanoate de propylèneglycol, et leurs mélanges.

Ledit ester peut être également choisi parmi les esters de synthèse notamment d'acide gras comme l'huile de purcellin, le myristate d'isopropyle, le palmitate d'éthyle, le stéarate d'octyle ; les esters hydroxylés comme le lactate d'isostéaryle, l'hydroxystéarate d'octyle, l'adipate de diisopropyle, les heptanoates, octanoates, décanoates d'alcool gras et leurs mélanges.

Par benzoate d'alkyle, on entend un ester répondant à la formule RCOOR',
dans laquelle R est un groupement phényle, substitué ou non substitué, et R' est un radical alkyle. En particulier le groupement phényle R du benzoate d'alkyle selon l'invention est dépourvu de fonction hydroxy.

L'isononanoate d'isononyle, le néopentanoate d'isostéaryle, le palmitate d'isopropyle, et leurs mélanges, conviennent tout particulièrement à la réalisation de l'invention.

Les esters hydrocarbonés courts sont avantageusement utilisés en une teneur suffisante pour permettre la solubilisation du glycérophospholipide non hydrogéné.

Cet ou ces ester(s) hydrocarboné(s) peuvent être présents dans les compositions cosmétiques conformes à la présente invention en une proportion allant de 5 à 95 %, en particulier de 10 à 80 % et plus particulièrement de 20 à 60 % en poids par rapport au poids total de la composition.

### ORGANOGEL

Par "gel", on entend signifier un système solide ou pseudo-solide mou comprenant au moins deux composants, dont l'un, liquide, constitue la partie majoritaire.

Au sens de la présente invention, on entend par "organogel", un système dont la composante liquide est une phase organique.

Les organogels peuvent être obtenus par addition d'une faible quantité d'eau à une composition cosmétique selon l'invention comprenant au moins un glycérophospholipide non hydrogéné en solution dans au moins un ester hydrocarboné court.

On entend par "faible quantité d'eau", au sens de la présente invention, la quantité d'eau minimum requise pour obtenir un gel à partir d'une solution de glycérophospholipide non hydrogéné dans un ester hydrocarboné court.

Le rapport pondéral ester hydrocarboné court - glycérophospholipide non hydrogéné nécessaire à l'obtention d'un organogel peut être déterminé à partir d'un mélange binaire d'ester(s) hydrocarboné(s) court(s) et de glycérophospholipide(s) non hydrogéné(s) selon le protocole suivant.

Des mélanges binaires glycérophospholipide non hydrogéné - ester hydrocarboné court sont obtenus en faisant varier la teneur en glycérophospholipide par rapport à la quantité d'ester court. A titre d'exemple, la teneur en glycérophospholipide non hydrogéné peut varier de 5 à 20 %. La solubilisation du glycérophospholipide non hydrogéné dans l'ester court est obtenue pour des concentrations variables en ester court, notamment, par chauffage du mélange à une température inférieure ou égale à 70 °C. Après solubilisation et retour à température ambiante (20-25 °C) des mélanges binaires, de l'eau ou un milieu aqueux est ajouté progressivement par agitation manuelle à chaque mélange, jusqu'à obtention d'un système apparaissant comme homogène. A titre d'exemple, de l'eau déminéralisée peut être utilisée lors de la réalisation de ce protocole. L'eau est rajoutée en des proportions variant de 1 à 15 % en poids par rapport au poids total du mélange.

Les teneurs en ester court et en glycérophospholipide non hydrogéné peuvent notamment être choisies pour que la teneur en eau à rajouter au mélange pour obtenir l'organogel ne dépasse pas 15 % en poids par rapport au poids total du mélange initial, et notamment soit inférieure ou égale à 10 %, notamment inférieure ou égale à 8 %, voire inférieure ou égale à 7 %, et encore inférieure ou égale à 5 %, voire inférieure ou égale à 3 %, voire inférieure ou égale à 2,5 %, ou encore inférieure ou égale à 2 %, et plus particulièrement inférieure ou égale à 1,5% en poids par rapport au poids total de la composition initiale.

De même, dans la composition selon l'invention, le rapport pondéral entre l'ester hydrocarboné court et le(s) glycérophospholipide(s) non hydrogéné(s) peut varier de 0,1 à 10, en particulier de 0,2 à 5, voire de 0,5 à 3.

Notamment, le rapport pondéral entre l'ester hydrocarboné court et la lécithine non hydrogénée utilisée à titre de source en glycérophospholipide que l'on mélange pour préparer la composition selon l'invention, peut varier de 0,1 à 10, notamment de 0,2 à 5, voire de 0,5 à 3.

La formation de l'organogel, une fois la composition cosmétique appliquée sur les matières kératiniques, peut se traduire par une augmentation de la viscosité de manière importante. Cette augmentation de viscosité améliore notamment la tenue du dépôt.

La viscosité des organogels susceptibles d'être obtenus à partir des mélanges binaires décrits ci-dessus, peut être déterminée à 20-25 °C à l'aide d'un viscosimètre rotatif de type METTLER RM 180. De manière avantageuse, lorsqu'un mélange binaire apte à la mise en oeuvre d'une composition cosmétique conforme à l'invention est sous forme d'organogel, il possède une viscosité allant de 30 à 50 poises.

La viscosité dynamique de la composition est mesurée avec un viscosimètre de type METTLER RM 180. L'appareil METTLER RM 180 (Rhéomat) peut être équipé de différents mobiles en fonction de l'ordre de grandeur de la viscosité que l'on veut mesurer. Pour une viscosité comprise entre 0,18 et 4,02 Pa.s, on équipe l'appareil d'un mobile 3. Pour une viscosité comprise entre 1 et 24 Pa.s, on équipe l'appareil d'un mobile 4, et pour une viscosité comprise entre 8 et 122 Pa.s, on équipe l'appareil d'un mobile 5. La viscosité est lue sur l'appareil en unités de déviation (UD). On se reporte ensuite à des abaques fournies avec l'appareil de mesure pour obtenir la valeur correspondante en poises.

La vitesse de rotation du mobile est de 200 tours/min.

A partir du moment où le mobile est mis en rotation, à une vitesse de rotation constante imposée (en l'espèce 200 tours/min), la valeur de viscosité de la composition peut varier au cours du temps. Des mesures sont prises à intervalle de temps réguliers jusqu'à ce qu'elles deviennent constantes. La valeur de la viscosité devenue constante au cours du temps est la valeur retenue comme étant la valeur de la viscosité dynamique de la composition selon l'invention. Selon le système de mesure imposé à l'appareil, la mesure de la viscosité est réalisée au terme de 10 minutes.

Selon un mode de réalisation de l'invention, un mélange binaire glycérophospholipide non hydrogéné ― ester hydrocarboné court convenant à la mise en oeuvre d'une composition cosmétique selon l'invention peut présenter, notamment, une aptitude à former un organogel possèdant une viscosité allant de 30 à 50 poises, en particulier de 35 à 50 poises, plus particulièrement de 40 à 50 poises, et en particulier de 45 à 50 poises.

### MILIEU PHYSIOLOGIQUEMENT ACCEPTABLE

Par "milieu physiologiquement acceptable", on désigne un milieu non toxique et susceptible d'être appliqué sur la peau, les lèvres ou les matières kératiniques d'êtres humains. Le milieu physiologiquement acceptable est généralement adapté à la nature du support sur lequel doit être appliquée la composition, ainsi qu'à l'aspect sous lequel la composition est destinée à être conditionnée.

Les compositions cosmétiques conformes à l'invention sont sous forme anhydre, c'est-à-dire qu'elles ne comporte pas une quantité d'eau suffisante pour induire par elle-même la formation d'un organogel à partir d'un mélange de glycérophospholipide non hydrogéné et d'ester hydrocarboné court, tel que défini précédemment.

On entend par "anhydre", au sens de la présente invention, une composition comprenant moins de 2,5 % en poids d'eau, notamment moins de 2 % en poids d'eau, voire moins de 1 % en poids d'eau, voire moins de 0,5 % en poids d'eau, ou encore moins de 0,1 % en poids d'eau par rapport au poids total de la composition.

Selon une variante de réalisation, la composition selon l'invention peut être exempte d'eau.

Elles peuvent se présenter, notamment, sous forme de gels huileux, de liquides huileux, de pâtes ou de sticks.

Selon un mode de réalisation, le milieu physiologiquement acceptable convenant à la mise en oeuvre de l'invention est, notamment, exempt de solvant polaire hydrophile. A titre de solvant polaire hydrophile susceptible d'être exclu des compositions selon l'invention, il peut être fait mention de l'eau, d'alcool tel que l'éthanol, l'alcool propylique, l'alcool isopropylique, l'hexanol et l'alcool benzylique ; les polyols, tels que le propylèneglycol, le polypropylèneglycol, le butylèneglycol, le maltitol, le sorbitol et la glycérine ; le panthénol dissout dans la glycérine ; les huiles aromatiques ; et leurs mélanges.

### Phase grasse

Les compositions cosmétiques conformes à la présente invention contiennent une phase grasse comprenant, outre au moins un ester hydrocarboné court, notamment des huiles et des corps gras solides à température ambiante (20 - 25 °C) et pression atmosphérique.

On entend par huile tout corps gras sous forme liquide à température ambiante (20 - 25 °C) et à pression atmosphérique. La phase grasse liquide peut, également, contenir outre des huiles, d'autres composés solubilisés dans les huiles tels que des agents gélifiants et/ou structurants, ainsi qu'exposé ci-après.

Bien que les esters hydrocarbonés courts, définis précédemment, puissent correspondre à la définition des huiles proposées ici, il est entendu que lorsqu'il est précisé que les compositions cosmétiques conformes à la présente invention peuvent contenir, en outre, une huile, cette huile est distincte d'un ester hydrocarboné court tel que défini précédemment.

La ou les huiles, hors esters hydrocarbonés courts, peuvent être présentes à raison de 0,1 à 90 % en poids, en particulier d'au moins 1 à 80 % en poids, plus particulièrement de 5 à 60 % en poids, notamment de 10 à 40 % en poids, voire de 20 à 30 % en poids, par rapport au poids total de la composition cosmétique selon l'invention.

Parmi les huiles, autres que les esters hydrocarbonés courts, convenant à la mise en oeuvre de l'invention, on distingue les huiles dans lesquelles le glycérophospholipide non hydrogéné est soluble et celles dans lesquelles il est au moins partiellement insoluble.

Les huiles convenant à la préparation des compositions cosmétiques selon l'invention peuvent être des huiles siliconées ou non.

Au sens de la présente invention, on entend par "huile siliconé", une huile comprenant au moins un atome de silicium, et notamment au moins un groupe Si-O.

On entend par "huile hydrocarbonée", une huile contenant principalement des atomes d'hydrogène et de carbone et éventuellement des atomes d'oxygène, d'azote, de soufre et/ou de phosphore.

Comme huile hydrocarbonée, on peut citer :
- les huiles hydrocarbonées d'origine animale,
- les huiles hydrocarbonées d'origine végétale telles que les esters de phytostéaryle, tels que l'oléate de phytostéaryle, l'isostéarate de physostéaryle et le glutamate de lauroyl/octyldodécyle/phytostéaryle (AJINOMOTO, ELDEW PS203), les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment des triglycérides héptanoïques ou octanoïques, les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de courge, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société STEARINERIES DUBOIS ou ceux vendus sous les dénominations MIGLYOL 810® , 812® et 818® par la société DYNAMIT NOBEL,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane et leurs mélanges, et en particulier le polyisobutène hydrogéné,
- les esters de polyols et les esters du pentaérythritol, comme le tétrahydroxystéarate/tétraisostéarate de dipentaérythritol,
- les esters de dimères diols et dimères diacides tels que les LUSPLAN DD-DA5® et LUSPLAN DD-DA7® , commercialisés par la société NIPPON FINE CHEMICAL et décrits dans la demande FR 0 302 809 déposée le 6 mars 2003, dont le contenu est incorporé dans la présente demande par référence.
- les alcools gras liquides à température ambiante à chaîne carbonée ramifiée et/ou insaturée ayant de 12 à 26 atomes de carbone comme le 2-octyldodécanol, l'alcool isostéarylique, l'alcool oléique, le 2-hexyldécanol, le 2-butyloctanol, et le 2-undécylpentadécanol,
- les acides gras supérieurs tels que l'acide oléique, l'acide linoléique, l'acide linolénique et leurs mélanges, et
- les carbonates de di-alkyle, les 2 chaînes alkyles pouvant être identiques ou différentes, tel que le carbonate de dicaprylyle commercialisé sous la dénomination CETIOL CC® , par COGNIS.

Les huiles de silicone convenant à la mise en oeuvre des compositions selon l'invention peuvent être les polydiméthylsiloxanes (PDMS), les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényltriméthicones, les phényldiméthicones, les phényltriméthylsiloxydiphénylsiloxanes, les diphényldiméthicones, les diphénylméthyldiphényltrisiloxanes, et les 2-phényléthyltriméthylsiloxysilicates, et leurs mélanges.

Parmi les huiles décrites précédemment, l'utilisation d'au moins une huile dans laquelle le glycérophospholipide non hydrogéné est au moins partiellement insoluble permet de concentrer le glycérophospholipide dans la phase huileuse hydrocarbonée dans laquelle il est soluble, notamment l'ester court, et de favoriser ainsi la formation de l'organogel.

Selon une variante de réalisation de l'invention, la composition conforme à l'invention peut comprendre en outre au moins une huile dans laquelle le glycérophospholipide non hydrogéné est au moins partiellement insoluble.

Selon une autre variante de réalisation de l'invention, l'huile dans laquelle le glycérophospholipide non hydrogéné est au moins partiellement insoluble est choisie parmi des huiles hydrocarbonées de haut poids moléculaire, des huiles de silicone, des huiles hydrocarbonées fluorées, et leurs mélanges.

Selon encore une variante de réalisation, l'huile dans laquelle le glycérophospholipide non hydrogéné est au moins partiellement insoluble est par exemple une huile hydrocarbonée de type polyisobutylène de haut poids moléculaire.

Selon une autre variante de réalisation, l'huile dans laquelle le glycérophospholipide non hydrogéné est au moins partiellement insoluble, est par exemple une huile de silicone phénylée, notamment la polyphényltriméthylsiloxydiméthylsiloxane commercialisée sous la référence BELSIM 1000® par la société WACKER.

L'huile dans laquelle le glycérophospholipide non hydrogéné est au moins partiellement insoluble peut être présente dans les compositions cosmétiques selon l'invention en une proportion variant de 0 à 60 % en poids, en particulier de 5 à 40 % en poids, et plus particulièrement de 10 à 25 % en poids, par rapport au poids total de la composition.

Selon une variante de réalisation, l'huile dans laquelle le glycérophospholipide non hydrogéné est au moins partiellement insoluble et ledit glycérophospholipide sont présents selon un rapport pondéral allant de 0,5 à 3, et notamment de 1 à 2,5, voire de 1,5 à 2.

La phase grasse liquide des compositions cosmétiques selon l'invention peut, le cas échéant, être épaissie, gélifiée ou structurée en y incorporant un agent gélifiant de phase grasse, comme par exemple des charges de type silice telles que définies ci-après, sous réserve que l'usage de ses composants soit compatible avec l'obtention d'un organogel dans les conditions requises selon l'invention.

### Cires et composés pâteux

Les compositions selon l'invention peuvent également comprendre au moins un composé choisi parmi les cires, les composés pâteux, et leurs mélanges.

Selon un mode de réalisation, les compositions selon l'invention comprenant un solvant polaire hydrophile, contiennent moins de 5 % de cire en poids par rapport au poids total de la composition. Notamment, ces compositions comprennent moins de 3 %, voire moins de 1 % en poids de cire par rapport au poids total de la composition.

Selon encore une autre variante, les compositions selon l'invention contenant un solvant polaire hydrophile sont notamment exemptes de cire.

Selon un autre mode de réalisation, les compositions selon l'invention exemptes de solvant polaire hydrophile peuvent comprendre au moins une cire, en une proportion notamment supérieure à 5 %.

La cire est solide à température ambiante (20-25 °C), à changement d'état solide/liquide réversible, ayant une température de fusion supérieure à 30 °C pouvant aller jusqu'à 200 °C, une dureté supérieure à 0,5 MPa et présentant à l'état solide une organisation cristalline anisotrope. Elle peut être hydrocarbonée, fluorée et/ou siliconée et être d'origine animale, végétale, minérale ou synthétique. Elle peut être choisie par exemple parmi la cire d'abeille, la cire de Carnauba, la cire de Candelilla, les cires de paraffine, l'huile de ricin hydrogénée, les cires synthétiques comme les cires de polyéthylène (de préférence de poids moléculaire compris entre 400 et 600) ou de Fischer-Tropsch, les cires de silicone comme les alkyl- ou alkoxy-diméthicone ayant de 16 à 45 atomes de carbone, les cérésines ou les ozokérites, comme par exemple les isoparaffines dont le point de fusion est inférieur à 40 °C, tel que l'EMW-0003, commercialisé par la société NIPPON SEIROU, les oligomères d'α-oléfine, tel que les polymères PERFORMA V® 825, 103 et 260, commercialisés par la société NEW PHASE TECHNOLOGIES ; les copolymères éthylène-propylène, tel que le PERFORMALENE® EP 700, et les cires microcristallines dont le point de fusion est supérieur à 85 °C, tel que les HI-MIC® 1070, 1080, 1090 et 3080, commercialisées par NIPPON SEIROU, et leurs mélanges.

De manière avantageuse, la cire utilisée dans les compositions cosmétiques conformes à l'invention, est par exemple, une cire de polyéthylène. La cire peut être également une cire microcristalline en C₂₀ - C₆₀, notamment la cire MICROWAX HW commercialisée par la société PARAMELT.

Selon un mode particulier de mise en oeuvre, la ou les cires utilisées dans les compositions cosmétique conformes à la présente invention peut ou peuvent être présentes en une teneur variant de 0 à 30 %, notamment de 5 à 20 %, voire de 8 à 17 %, en poids par rapport au poids total de la composition.

Les compositions cosmétiques conformes à la présente invention peuvent également comprendre au moins un composé pâteux.

Par "pâteux" au sens de la présente invention, on entend un composé gras à changement d'état solide/liquide réversible, et comportant à la température de 23°C une fraction liquide et une fraction solide. On entend également par pâteux, le polylaurate de vinyle.

Le composé pâteux au sens de l'invention présente avantageusement une dureté à 20 °C allant de 0,001 à 0,5 MPa, de préférence de 0,002 à 0,4 MPa.

Parmi les composés pâteux susceptibles d'être utilisés dans la composition selon l'invention, on peut citer les lanolines et les dérivés de lanoline comme les lanolines acétylées, les lanolines oxypropylénées ou le lanolate d'isopropyle, et leurs mélanges. On peut également utiliser des esters d'acides ou d'alcools gras, notamment ceux ayant 20 à 65 atomes de carbone comme le citrate de tri-isostéaryle ou de cétyle ; le propionate d'arachidyle ; le polylaurate de vinyle ; les esters du cholestérol comme les triglycérides d'origine végétale tels que les huiles végétales hydrogénées, les polyesters visqueux et leurs mélanges. Comme triglycéride d'origine végétale, on peut citer les dérivés d'huile de ricin hydrogénée, tels que le THIXINR® de RHEOX, et les coco-glycérides hydrogènes.

Avantageusement, le corps gras pâteux peut être un mélange de triglycérides d'acide laurique/palmitique/cétylique/stéarique (50/20/10/10), notamment celui commercialisé par la société JASOL sous la référence SOFTISAN 100® .

On peut également citer les polyesters résultant de l'estérification d'un acide carboxylique et d'un ester acide hydroxycarboxylique aliphatique. Par exemple, le RISOCAST® DA-L (ester issu de la réaction d'estérification de l'huile de ricin hydrogéné avec de l'acide dilinoléïque dans des proportions de 2 pour 1) et le RISOCAST® DA-H (ester résultant de l'estérification de l'huile de ricin hydrogénée avec de l'acide isostéarique dans des proportions de 4 pour 3) commercialisés par la société japonaise KOKYU ALCOHOL KOGYO.

On peut aussi citer les composés pâteux siliconés tels que des polydiméthylsiloxanes (PDMS) de haut poids moléculaire et en particulier ceux ayant des chaînes pendantes du type alkyle ou alcoxy ayant de 8 à 24 atomes de carbone, et un point de fusion de 20-55°C, comme les stéaryl diméthicones notamment ceux vendus par la société DOW CORNING sous les noms commerciaux de DC2503® et DC25514® et leurs mélanges.

Selon une variante de réalisation, le composé pâteux peut être présent dans les compositions cosmétiques selon l'invention selon une proportion variant de 0 à 30 % en poids, et notamment de 10 à 20 % en poids par rapport au poids total de la composition.

### ADDITIFS

Les compositions cométiques conformes à l'invention peuvent également comprendre tout additif usuellement utilisé dans le domaine concerné sous réserve que ces additifs n'altèrent pas la propriété des compositions selon l'invention à former un organogel par mise en contact avec une phase aqueuse.

Les additifs susceptibles de convenir à la mise en oeuvre de l'invention peuvent être choisis notamment parmi des matières colorantes, comme les nacres et les pigments, des charges, des antioxydants, des agents filmogènes, et le cas échéant des auxiliaires de filmification, des agents gélifiants, des huiles essentielles, des conservateurs, des parfums, des agents hydratants, des agents antiseptiques, des vitamines telles que les vitamines B3, E et leurs dérivés, des agents protecteurs contre les UV, des neutralisants, des tensioactifs, et leurs mélanges.

Bien entendu l'homme du métier veillera également à choisir les éventuels additifs complémentaires et/ou leur quantité de telle manière que, outre l'aptitude à former un organogel, les propriétés avantageuses de la composition selon l'invention, à savoir tenue, brillance, aspect non collant, couvrance et non migration notamment ne soient pas, ou substantiellement pas, altérées par l'adjonction envisagée.

### Matières colorantes

Selon un mode de réalisation, la composition selon l'invention peut contenir en outre au moins une matière colorante, organique ou inorganique, notamment de type pigments ou nacres.

Selon un autre mode de réalisation, la composition selon l'invention peut contenir en outre au moins une matière colorante choisie parmi les colorants lipophiles, les colorants hydrophiles, les pigments, les nacres, les matériaux à effet optique spécifique, et leurs mélanges.

Cette matière colorante peut être présente à raison de 0,01 à 50 % en poids par rapport au poids total de la composition, en particulier de 0,5 à 40 % et plus particulièrement de 5 à 25 %, et notamment de 0,01 à 20 %, et en particulier de 0,1 à 10 %, voire de 2 à 5 % en poids par rapport au poids total de la composition.

Par "pigments", il faut comprendre des particules blanches ou colorées, minérales ou organiques, insolubles dans une solution aqueuse, destinées à colorer et/ou opacifier le film résultant.

Les pigments peuvent être présents à raison de 0,01 à 20 % en poids, notamment de 0,01 à 5 % en poids, et en particulier de 0,02 à 7 % en poids, par rapport au poids total de la composition cosmétique.

Comme pigments minéraux utilisables dans l'invention, on peut citer les oxydes de titane, de zirconium ou de cérium ainsi que les oxydes de zinc, de fer ou de chrome, le bleu ferrique, le violet de manganèse, le bleu outremer et l'hydrate de chrome.

Il peut également s'agir de pigment ayant une structure qui peut être par exemple de type séricite/oxyde de fer brun/dioxyde de titane/silice. Un tel pigment est commercialisé par exemple sous la référence COVERLEAF® NS ou JS par la société CHEMICALS AND CATALYSTS et présente un rapport de contraste voisin de 30.

La matière colorante peut encore comporter un pigment ayant une structure qui peut être par exemple de type microsphères de silice contenant de l'oxyde de fer. Un exemple de pigment présentant cette structure est celui commercialisé par la société MIYOSHI sous la référence PC BALL® PC-LL-100 P, ce pigment étant constitué de microsphères de silice contenant de l'oxyde de fer jaune.

Parmi les pigments organiques utilisables dans l'invention, on peut citer le noir de carbone, les pigments de type D & C, les laques à base de carmin de cochenille, de baryum, strontium, calcium, aluminium ou encore les dicéto pyrrolopyrrole (DPP) décrits dans les documents EP-A-542669, EP-A-787730, EP-A-787731 et WO-A- 96/08537.

Par "nacres", il faut comprendre des particules colorées de toute forme, irisées ou non, notamment produites par certains mollusques dans leur coquille ou bien synthétisées et qui présentent un effet de couleur par interférence optique.

Les nacres peuvent être choisies parmi les pigments nacrés tels que le mica titane recouvert avec un oxyde de fer, le mica recouvert d'oxychlorure de bismuth, le mica titane recouvert avec de l'oxyde de chrome, le mica titane recouvert avec un colorant organique ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

On peut également citer, à titre d'exemple de nacres, le mica naturel recouvert d'oxyde de titane, d'oxyde de fer, de pigment naturel ou d'oxychlorure de bismuth.

Parmi les nacres disponibles sur le marché, on peut citer les nacres TIMICA® , FLAMENCO® et DUOCHROME® (sur base de mica) commercialisées par la société ENGELHARD, les nacres TIMIRON® commercialisées par la société MERCK, les nacres sur base de mica PRESTIGE® commercialisées par la société ECKART et les nacres sur base de mica synthétique SUNSHINE® commercialisées par la société SUN CHEMICAL.

Les nacres peuvent plus particulièrement posséder une couleur ou un reflet jaune, rose, rouge, bronze, orangé, brun, or et/ou cuivré.

Les pigments peuvent être ou non enrobés superficiellement, en particulier traités en surface par des silicones, des acides aminés, des dérivés fluorés ou toute autre substance favorisant la dispersion et la compatibilité du pigment dans la composition.

Avantageusement, les pigments utilisés dans les compositions conformes à l'invention peuvent également être enrobés superficiellement par un revêtement de lécithine. Ce revêtement peut être obtenu par mise en contact d'une solution de pigment avec une solution de lécithine, en présence de sels de métaux di- ou trivalents. Pour obtenir ce revêtement, de la lécithine hydrogénée ou non hydrogénée peut être utilisée.

La composition cosmétique selon l'invention peut comporter également des colorants hydrosolubles ou liposolubles en une teneur allant de 0,01 à 10 % en poids, notamment allant de 0,01 à 5 % en poids par rapport au poids total de la composition cosmétique.

Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine.

Lorsque les compositions cosmétiques selon l'invention comportent un colorant hydrosoluble, ce dernier peut être présent dans la composition à l'état dispersé.

La composition cosmétique selon l'invention peut également contenir au moins un matériau à effet optique spécifique.

Cet effet est différent d'un simple effet de teinte conventionnel, c'est-à-dire unifié et stabilisé tel que produit par les matières colorantes classiques comme par exemple les pigments monochromatiques. Au sens de l'invention, "stabilisé" signifie dénué d'effet de variabilité de la couleur avec l'angle d'observation ou encore en réponse à un changement de température.

Par exemple, ce matériau peut être choisi parmi les particules à reflet métallique, les agents de coloration goniochromatiques, les pigments diffractants, les agents thermochromes, les agents azurants optiques, ainsi que les fibres, notamment interférentielles. Bien entendu, ces différents matériaux peuvent être associés de manière à procurer la manifestation simultanée de deux effets, voire d'un nouvel effet conforme à l'invention.

Les particules à reflet métallique utilisables dans l'invention sont en particulier choisies parmi :
- les particules d'au moins un métal et/ou d'au moins un dérivé métallique,
- les particules comportant un substrat, organique ou minéral, monomatière ou multimatériaux, recouvert au moins partiellement par au moins une couche à reflet métallique comprenant au moins un métal et/ou au moins un dérivé métallique, et
- les mélanges desdites particules.

Parmi les métaux pouvant être présents dans lesdites particules, on peut citer par exemple Ag, Au, Cu, Al, Ni, Sn, Mg, Cr, Mo, Ti, Zr, Pt, Va, Rb, W, Zn, Ge, Te, Se et leurs mélanges ou alliages. Ag, Au, Cu, Al, Zn, Ni, Mo, Cr, et leurs mélanges ou alliages (par exemple les bronzes et les laitons) sont des métaux préférés.

Par "dérivés métalliques", on entend désigner des composés dérivés de métaux notamment des oxydes, des fluorures, des chlorures et des sulfures

A titre illustratif de ces particules, on peut citer des particules d'aluminium, telles que celles commercialisées sous les dénominations STARBRITE 1200 EAC® par la société SIBERLINE et METALURE® par la société ECKART.

On peut également citer les poudres métalliques de cuivre ou des mélanges d'alliage telles les références 2844 commercialisées par la société RADIUM BRONZE, les pigments métalliques comme l'aluminium ou le bronze, telles que celles commercialisées sous les dénominations ROTOSAFE® 700 de la société ECKART, les particules d'aluminium enrobé de silice commercialisées sous la dénomination VISIONAIRE BRIGHT SILVER® de la société ECKART et les particules d'alliage métallique comme des poudres de bronze (alliage cuivre et zinc) enrobé de silice commercialisées sous la dénomination de VISIONAIRE BRIGHT NATURAL GOLD® de la société ECKART.

Il peut encore s'agir de particules comportant un substrat de verre comme celles commercialisées par la société NIPPON SHEET GLASS sous les dénominations MICROGLASS METASHINE® .

L'agent de coloration goniochromatique peut être choisi par exemple parmi les structures multicouches interférentielles et les agents de coloration à cristaux liquides.

Des exemples de structures multicouche interférentielles symétriques utilisables dans des compositions réalisées conformément à l'invention sont par exemple les structures suivantes : Al/SiO₂/Al/SiO₂/Al, des pigments ayant cette structure étant commercialisés par la société DUPONT DE NEMOURS ; Cr/MgF₂/Al/MgF₂/Cr, des pigments ayant cette structure étant commercialisés sous la dénomination CHROMAFLAIR par la société FLEX ; MoS₂/SiO₂/Al/SiO₂/MoS₂ ; Fe₂O₃/SiO₂/Al/SiO₂/Fe₂O₃, et Fe₂O₃/SiO₂/Fe₂O₃/SiO₂/Fe₂O₃, des pigments ayant ces structures étant commercialisés sous la dénomination SICOPEARL par la société BASF ; MoS₂/SiO₂/mica-oxyde/SiO₂/MoS₂ ; Fe₂O₃/SiO₂/mica-oxyde/SiO₂/Fe₂O₃ ; TiO₂/SiO₂/TiO₂ et TiO₂/Al₂O₃/TiO₂ ; SnO/TiO₂/SiO₂/TiO₂/SnO ; Fe₂O₃/SiO₂/Fe₂O₃ ; SnO/mica/TiO₂/SiO₂/TiO₂/mica/SnO, des pigments ayant ces structures étant commercialisés sous la dénomination XIRONA® par la société MERCK (Darmstadt). A titre d'exemple, ces pigments peuvent être les pigments de structure silice/oxyde de titane/oxyde d'étain commercialisés sous le nom XIRONA MAGIC® par la société MERCK, les pigments de structure silice/oxyde de fer brun commercialisés sous le nom XIRONA INDIAN SUMMER® par la société MERCK et les pigments de structure silice/oxyde de titane/mica/oxyde d'étain commercialisés sous le nom XIRONA CARRIBEAN BLUE® par la société MERCK. On peut encore citer les pigments INFINITE COLORS® de la société SHISEIDO. Selon l'épaisseur et la nature des différentes couches, on obtient différents effets. Ainsi, avec la structure Fe₂O₃/SiO₂/Al/ SiO₂/Fe₂O₃ on passe du doré-vert au gris-rouge pour des couches de SiO₂ de 320 à 350 nm ; du rouge au doré pour des couches de SiO₂ de 380 à 400 nm ; du violet au vert pour des couches de SiO₂ de 410 à 420 nm ; du cuivre au rouge pour des couches de SiO₂ de 430 à 440 nm.

On peut citer, à titre d'exemple de pigments à structure multicouche polymérique, ceux commercialisés par la société 3M sous la dénomination COLOR GLITTER.

Comme particules goniochromatiques à cristaux liquides, on peut utiliser par exemple celles vendues par la société CHENIX ainsi que celle commercialisées sous la dénomination HELICONE® HC par la société WACKER.

### Charges

De manière avantageuse, les compositions cosmétiques conformes à l'invention peuvent également comprendre au moins une charge, de nature organique ou minérale.

Par "charge", il faut comprendre les particules incolores ou blanches, solides de toutes formes, qui se présentent sous une forme insoluble et dispersée dans le milieu de la composition. De nature minérale ou organique, elles permettent de conférer du corps ou de la rigidité à la composition, et/ou de la douceur, de la matité et de l'uniformité au maquillage.

Les charges utilisées dans les compositions selon la présente invention peuvent être de formes lamellaires, globulaires, sphériques, de fibres ou de toute autre forme intermédiaire entre ces formes définies.

Les charges selon l'invention peuvent être ou non enrobées superficiellement, en particulier elles peuvent être traitées en surface par des silicones, des acides aminés, des dérivés fluorés ou toute autre substance favorisant la dispersion et la compatibilité de la charge dans la composition.

Au sens de la présente invention, les termes "charges minérales" et "charges inorganiques" sont utilisés de manière interchangeable.

Parmi les charges minérales utilisables dans les compositions selon l'invention, on peut citer le talc, le mica, la silice, le siloxysilicate de triméthyle, le kaolin, la bentone, l'hydroxyapatite, le nitrure de bore, les microsphères de silice creuses (SILICE BEADS® de MAPRECOS), les microcapsules de verre ou de céramique, les charges à base de silice comme l'AEROSIL® 200, l'AEROSIL® 300 ; le SUNSPHARE® L-31, le SUNPHARE® H-31 commercialisés par ASAHI GLASS ; le CHEMICELEN® commercialisé par ASAHI CHEMICAL ; les composites de silice et de dioxyde de titane comme la série TSG commercialisée par NIPPON SHEET GLASS, et leurs mélanges.

Parmi les charges organiques utilisables dans les compositions selon l'invention on peut citer les poudres de polyamide (NYLON® ORGASOL de chez ATOCHEM), de poly-b-alanine et polyéthylène, les poudres de polytétrafluoroéthylène (TEFLON® ), la lauroy-lysine, l'amidon, les poudres de polymères de tétrafluoroéthylène, les microsphères creuses de polymères telles l'EXPANCEL® (NOBEL INDUSTRIE), le carbonate de calcium précipité, le carbonate et l'hydrogéno-carbonate de magnésium, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium, le POLYPORE® L 200 (CHEMDAL CORPORATION), les microbilles de résine de silicone (TOSPEARL® de TOSHIBA, par exemple), les poudres de poyuréthanne, en particulier les poudres de polyuréthanne réticulé comprenant un copolymère, ledit copolymère comprenant du triméthylol hexyllactone. En particulier, il peut s'agir d'un polymère d'hexaméthylène di-isocyanate/triméthylol hexyllactone. De telles particules sont notamment disponibles dans le commerce, par exemple sous la dénomination de PLASTIC POWDER D-400® ou PLASTIC POWDER D-800® de la société TOSHIKI, et leurs mélanges.

Les charges peuvent être présentes dans les compositions cosmétiques conformes à l'invention à raison de 0,001 à 60 %, de préférence 0,5 à 35 %, notamment de 0,5 à 20 %, voire de 1 à 10 % en poids par rapport au poids total de la composition.

La charge peut être par exemple une charge dont la granulométrie moyenne est inférieure à 100 µm, notamment comprise entre 1 et 50 µm, par exemple entre 4 et 20 µm.

La présente invention a encore pour objet un procédé cosmétique de soin, de maquillage et/ou de traitement des matières kératiniques comprenant au moins l'étape d'application sur ces matières kératiniques d'une composition cosmétique telle que définie précédemment.

La surface à maquiller peut être, préalablement à l'application de la composition cosmétique selon l'invention, préparée par application d'une composition comprenant un milieu aqueux.

Selon une autre variante de réalisation, cette phase aqueuse peut être ajoutée après application de la composition cosmétique sur la surface à maquiller.

Le milieu aqueux peut être de la salive, des larmes, de la sueur. Il peut également être de l'eau minérale, de l'eau de source, de l'eau du robinet, de l'eau de mer, une solution aqueuse, une solution hydroalcoolique, voire une émulsion, notamment une émulsion huile-dans-eau. Le milieu aqueux peut également contenir au moins un composé choisi parmi un colorant, un parfum, un actif cosmétique et/ou dermatologique, et leurs mélanges. Le milieu aqueux peut être appliqué, par exemple, par vaporisation ou mouillage.

Selon encore une autre variante de réalisation, la composition conforme à l'invention peut être fournie indépendamment ou en combinaison avec un milieu aqueux.

Selon encore une autre variante de réalisation, lorsque le milieu aqueux est fourni en combinaison avec une composition conforme à l'invention, celui-ci peut être conditionné en récipient monodose ou multidoses.

Ainsi, une composition selon l'invention peut être fournie sous la forme d'un kit de soin et/ou de maquillage comportant au moins une première composition comprenant de l'eau, et au moins une deuxième composition anhydre contenant, dans un milieu physiologiquement acceptable, au moins une phase grasse comprenant au moins un ester hydrocarboné court et au moins un glycérophospholipide non hydrogéné.

La première composition comprend de l'eau en quantité suffisante, pour que la deuxième composition forme un organogel lorsqu'elle est mise en contact avec la première composition.

La première composition comprenant de l'eau peut être, par exemple, un milieu aqueux tel que défini précédemment.

Selon encore un autre mode de réalisation, la présente invention a encore pour objet un procédé de soin et/ou de maquillage des matières kératiniques comprenant au moins une étape d'application, l'une sur l'autre, d'une première composition comprenant de l'eau, et d'une deuxième composition anhydre contenant, dans un milieu physiologiquement acceptable, au moins une phase grasse comprenant au moins un ester hydrocarboné court et au moins un glycérophospholipide non hydrogéné.

Dans le procédé précédent, l'ordre d'application de la première et de la deuxième composition peut être indifférent.

Selon une variante de réalisation, le glycérophospholipide non hydrogéné est introduit dans la composition conforme à l'invention sous la forme d'une lécithine non hydrogénée à raison d'environ 10 % en poids par rapport au poids total de la composition. Cette lécithine peut être une lécithine contenant au moins 95 % en poids de glycérophospholipide non hydrogéné par rapport au poids total de la lécithine. Ce glycérophospholipide non hydrogéné peut être de la phosphatidylcholine non hydrogénée.

Selon une autre variante, la composition cosmétique conforme à la présente invention comprend en tant que glycérophospholipide non hydrogéné, la phosphatidylcholine non hydrogénée, et en tant qu'ester court, au moins l'isononanoate d'isononyle.

Selon une autre variante, la composition cosmétique selon l'invention comprend en tant que glycérophospholipide non hydrogéné, la phosphatidylcholine non hydrogénée, et en tant que cire, une cire microcristalline.

Selon encore une autre variante, la composition cosmétique selon la présente invention, comprend en tant que glycérophospholipide non hydrogéné, la phosphatidylcholine non hydrogénée, et en tant que composé pâteux, un mélange de triglycérides d'acides laurique/palmitique/cétylique/stéarique (50/20/10/10).

La composition cosmétique selon l'invention peut, notamment, se présenter sous la forme d'un produit de maquillage des lèvres, en particulier sous la forme d'un rouge à lèvres ou d'un baume à lèvres.

Les exemples de compositions ci-après sont donnés à titre illustratif et sans caractère limitatif de l'invention.

### EXEMPLE I : MELANGE BINAIRE

Des mélanges binaires de lécithine non hydrogénée à 20 %, comprenant au moins 95 % en poids de glycérophospholipide non hydrogéné par rapport au poids total de la lécithine (EMULMETIK 930), et d'ester hydrocarboné court à 80 % ont été préparés par agitation et chauffage jusqu'à environ 70 °C, jusqu'à obtention de la solubilisation de la lécithine.

Après refroidissement à température ambiante, de l'eau est rajoutée au mélange, sous agitation, à raison de 1 % par rapport au poids total du mélange binaire.

L'aspect des compositions est observé après solubilisation de la lécithine et 24 heures après adjonction d'eau. Les résultats sont résumés dans le tableau (I) ci-dessous.

Les résultats montrent qu'un mélange binaire de lécithine non hydrogénée et d'ester hydrocarboné court, en proportions respectivement de 20 % et 80 %, est susceptible de former un organogel par adjonction d'une faible proportion d'eau.

| **Ester court** | **Aspect du mélange binaire après solubilisation** | **Aspect du mélange 24h après adjonction d'1 % d'eau** | **Conclusion** |
|---|---|---|---|
| Myristate d'isopropyle | Liquide limpide jaune | Gel épais jaune transparent | Organogel |
| Palmitate d'isopropyle | Liquide limpide jaune | Liquide épais jaune transparent | Organogel |
| Isononanoate d'isononyle | Liquide limpide jaune clair | Liquide épais jaune transparent | Organogel |

### EXEMPLE II : ROUGE A LEVRES

**Tableau II**

| **Composition témoin** | | **Composition A** | |
|---|---|---|---|
| **% en poids** | | **% en poids** | |
| Malate de di-isostearyle | 22,71 | Isononanoate d'isononyle | 18,92 |
| Phényltriméthylsiloxitrisiloxane (viscosité : 20 cSt) | 18,17 | Phényltriméthylsiloxitrisiloxane (viscosité :20 cSt) | 15,69 |
| Polyphényltriméthylsiloxidiméthylsiloxane (viscosité : 1000 cSt) | 27,26 | Polyphényltriméthylsiloxidiméthylsiloxane (viscosité : 1000 cSt) | 23,53 |
| | - | Lécithine non hydrogénée (EMULMETIK 930) | 10,00 |
| Cire microcristalline | 10,00 | Cire microcristalline | 10,00 |
| Polydiméthylsiloxane α-ω alkylé en C30/C45 | 2,50 | Polydiméthylsiloxane α-ω alkylé en C30/C45 | 2,50 |
| Triglycéride d'acide laurique/palmitique/cétylique/stéarique (50/20/10/10) | 10,00 | Triglycéride d'acide laurique/palmitique/cétylique/stéarique (50/20/10/10) | 10,00 |
| Malate de di-isostearyle | 5,00 | Isononanoate d'isononyle | 5,00 |
| Eosine | 0,06 | Eosine | 0,06 |
| Sel de calcium du rouge lithol B | 0,26 | Sel de calcium du rouge lithol B | 0,26 |
| Oxyde de fer noir | 0,09 | Oxyde de fer noir | 0,09 |
| Oxyde de fer brun | 2,15 | Oxyde de fer brun | 2,15 |
| Mica- Oxyde de titane (67,5/32,5) | 1,80 | Mica- Oxyde de titane (67,5/32,5) | 1,80 |

### Mode opératoire

Deux formulations de rouge à lèvres sont préparées, une composition témoin et une composition conforme à l'invention, la composition A.

Une première phase huileuse est préparée en mélangeant à chaud (à environ 70 °C) les huiles, une fraction de l'ester court et la lécithine non hydrogénée (lorsqu'ils sont présents, à savoir dans la composition A).

Une seconde phase huileuse est préparée en mélangeant à une autre fraction de l'ester court les colorants.

Les cires et les composés pâteux sont ensuite ajoutés au mélange.

Le mélange ainsi obtenu est ensuite coulé dans un moule de rouge à lèvres et laissé refroidir jusqu'à l'obtention d'une composition solide.

Le rouge à lèvres formulé sur la base d'une composition conforme à la présente invention (composition A) présente une sensation d'effet collant nettement inférieur à celui de la composition témoin, tout en ayant une tenue améliorée et une brillance satisfaisante.

## Revendications

1. Composition cosmétique anhydre contenant, dans un milieu physiologiquement acceptable, au moins une phase grasse comprenant au moins un ester hydrocarboné court et au moins un glycérophospholipide non hydrogéné, ladite composition étant exempte de solvant polaire hydrophile.

2. Composition cosmétique anhydre contenant, dans un milieu physiologiquement acceptable, au moins une phase grasse comprenant au moins un ester hydrocarboné court et au moins un glycérophospholipide non hydrogéné, ladite composition comprenant moins de 5 % en poids de cire par rapport au poids total de la composition.

3. Composition cosmétique anhydre contenant, dans un milieu physiologiquement acceptable, au moins une phase grasse comprenant au moins un ester hydrocarboné court et au moins un glycérophospholipide non hydrogéné, ledit ester hydrocarboné court et ledit glycérophospholipide non hydrogéné y étant respectivement présents en des teneurs correspondant à celles requises dans un mélange binaire constitué desdits glycérophospholipide non hydrogéné et ester hydrocarboné court pour lui conférer une aptitude à former un organogel par mise en contact avec un milieu aqueux.

4. Composition selon l'une quelconque des revendications précédentes, **caractérisée en ce que** le glycérophospholipide non hydrogéné et l'ester hydrocarboné court y sont présents respectivement en des teneurs correspondant à celles requises dans un mélange binaire constitués desdits glycérophospholipide non hydrogéné et ester hydrocarboné court pour lui conférer une aptitude à former un organogel possédant une viscosité allant de 30 à 50 poises, en particulier de 35 à 50 poises, plus particulièrement de 40 à 50 poises, et en particulier de 45 à 50 poises.

5. Composition cosmétique selon l'une quelconque des revendications précédentes, **caractérisée en ce que** l'ester court et le glycérophospholipide non hydrogéné sont présents selon un rapport pondéral variant de 0,1 à 10, en particulier de 0,2 à 5, voire de 0,5 à 3.

6. Composition selon l'une quelconque des revendications précédentes, **caractérisée en ce que** ledit glycérophospholipide non hydrogéné est présent en une teneur d'au moins 5 %, d'au moins 10 %, en particulier d'au moins 15 %, et plus particulièrement d'au moins 20 % en poids, par rapport au poids total de la composition.

7. Composition selon l'une quelconque des revendications précédentes, **caractérisée en ce que** le glycérophospholipide non hydrogéné est choisi parmi la phosphatidylcholine non hydrogénée, la phosphatidyléthanolamine non hydrogénée, la phosphatidylsérine non hydrogénée, et leurs mélanges.

8. Composition cosmétique selon l'une quelconque des revendications précédentes, **caractérisée en ce que** le glycérophospholipide non hydrogéné est introduit dans ladite composition sous la forme d'une lécithine non hydrogénée.

9. Composition selon la revendication précédente, **caractérisée en ce que** la lécithine non hydrogénée est choisie parmi les lécithines issues du soja, du tournesol, de l'oeuf, et leurs mélanges.

10. Composition cosmétique selon la revendication 8 ou 9, **caractérisée en ce que** ladite lécithine non hydrogénée comprend au moins 45 %, en particulier au moins 65 % en poids, en particulier au moins 75 % en poids, en particulier au moins 85 % en poids, et plus particulièrement au moins 95 % en poids de glycérophospholipide non hydrogéné par rapport au poids total de la lécithine.

11. Composition selon l'une quelconque des revendications 8 à 10, **caractérisée en ce que** la lécithine non hydrogénée est présente à raison d'au moins 5 %, d'au moins 10 % en poids, en particulier au moins 15 % en poids, et plus particulièrement au moins 20 % en poids par rapport au poids total de la composition.

12. Composition selon l'une quelconque des revendications précédentes, **caractérisée en ce que** ledit ester hydrocarboné court est choisi parmi les monoesters et les diesters non hydroxylés, lesdits monoesters et diesters comprenant moins de 40 atomes de carbone.

13. Composition selon l'une quelconque des revendications précédentes, **caractérisée en ce que** ledit ester hydrocarboné court est choisi parmi l'isononanoate d'isononyle, le néopentanoate d'isostéaryle, le palmitate d'isopropyle, et leurs mélanges.

14. Composition selon l'une quelconque des revendications précédentes, **caractérisée en ce que** ledit ester hydrocarboné court est présent en une proportion allant de 5 à 95 %, en particulier de 10 à 80 %, et plus particulièrement allant de 20 à 60 %, en poids par rapport au poids total de la composition.

15. Composition selon l'une quelconque des revendications précédentes, **caractérisée en ce qu'**elle comprend en outre au moins une huile dans laquelle ledit glycérophospholipide non hydrogéné est au moins partiellement insoluble.

16. Composition selon la revendication précédente, **caractérisée en ce que** ladite huile est choisie parmi des huiles hydrocarbonées de haut poids moléculaire, des huiles de silicone, des huiles hydrocarbonées fluorées, et leurs mélanges.

17. Composition selon la revendication 15 ou 16, **caractérisée en ce que** ladite huile est présente en une proportion variant de 0 à 60 % en poids, en particulier de 5 à 40 % en poids, et plus particulièrement de 10 à 25 % en poids par rapport au poids total de la composition.

18. Composition selon l'une quelconque des revendications 15 à 17, **caractérisée en ce que** ladite huile et ledit glycérophospholipide non hydrogéné sont présents selon un rapport pondéral allant de 0,5 à 3, notamment de 1 à 2,5, voire de 1,5 à 2.

19. Composition selon l'une quelconque des revendications 15 à 18, **caractérisée en ce que** ladite huile est une huile de silicone phénylée.

20. Composition selon l'une quelconque des revendications précédentes, **caractérisée en ce qu'**elle comprend en outre au moins un composé choisi parmi les corps gras pâteux, les cires, et leurs mélanges.

21. Composition selon la revendication précédente, **caractérisée en ce que** ledit composé est une cire de polyéthylène.

22. Composition selon la revendication 20, **caractérisée en ce que** ledit composé est un corps pâteux choisi parmi la lanoline et ses dérivés, des esters d'acides ou d'alcools gras, des polydiméthylsiloxanes de haut poids moléculaire et leurs mélanges.

23. Composition selon l'une quelconque des revendications précédentes, **caractérisée en ce qu'**elle comprend en outre au moins un additif choisi parmi des charges, des pigments, des antioxydants, des huiles essentielles, des conservateurs, des neutralisants, des parfums, des tensioactifs, et leurs mélanges.

24. Composition selon l'une quelconque des revendications précédentes, **caractérisée en ce qu'**elle se présente sous la forme d'un rouge à lèvres.

25. Procédé cosmétique de soin, de maquillage et/ou de traitement des matières kératiniques comprenant au moins l'étape d'application sur ces matières kératiniques d'une composition cosmétique conforme à l'une quelconque des revendications précédentes.

26. Support synthétique maquillé, comprenant sur tout ou partie de sa surface au moins une couche d'une composition cosmétique conforme à l'une quelconque des revendications précédentes.

27. Utilisation cosmétique d'au moins un glycérophospholipide non hydrogéné en association avec au moins un ester hydrocarboné court pour conférer à une composition cosmétique une aptitude à former un organogel.

28. Utilisation selon la revendication 27, **caractérisée en ce que** ledit glycérophospholipide non hydrogéné est tel que défini selon l'une quelconque des revendications 3 à 11.

29. Utilisation selon la revendication 27 ou 28, **caractérisée en ce que** ledit ester hydrocarboné court est tel que défini selon l'une quelconque des revendications 12 à 14.

30. Kit de soin et/ou de maquillage comportant :
- au moins une première composition comprenant de l'eau, et
- au moins une deuxième composition anhydre contenant, dans un milieu physiologiquement acceptable, au moins une phase grasse comprenant au moins un ester hydrocarboné court et au moins un glycérophospholipide non hydrogéné.

31. Kit selon la revendication précédente, **caractérisé en ce que** la première composition comprend de l'eau en quantité suffisante, pour que la deuxième composition forme un organogel lorsqu'elle est mise en contact avec la première composition.

32. Kit selon la revendication 30 ou 31, **caractérisé en ce que** la première composition est une solution aqueuse ou une émulsion huile-dans-eau.

33. Kit selon l'une quelconque des revendications 30 à 32, **caractérisé en ce que** la première composition comprend au moins un actif cosmétique et/ou dermatologique.

34. Kit selon l'une quelconque des revendications 30 ou 33, **caractérisé en ce que** la première composition est conditionnée en récipient mono ou multidoses.

35. Procédé de maquillage des matières kératiniques, comprenant au moins une étape d'application l'une sur l'autre :
- d'une première composition comprenant de l'eau, et
- d'une deuxième composition anhydre comprenant, dans un milieu physiologiquement acceptable, au moins une phase grasse comprenant au moins un ester hydrocarboné court et au moins un glycérophospholipide non hydrogéné.
